# EUROPÄISCHE PATENTANMELDUNG

(11) **EP 0 808 901 A2**
(43) Veröffentlichungstag der Anmeldung: **26.11.1997**
(21) Anmeldenummer: 97890095.9
(22) Anmeldetag: 23.05.1997
(51) Int. Cl.: C12N 15/12, A61K 38/37, C07K 14/755, C07K 16/36

(54) **Pharmazeutische Präparation mit Faktor VIII-Prokoagulations-aktivität und vWF-Bindungs-aktivität**

(30) Priorität: 24.05.1996 AT 921/96
(71) Anmelder: IMMUNO AG, 1221 Wien (AT)
(72) Erfinder: Voorberg, Jan, NL-1566 RH Assendelft (NL)
(74) Vertreter: Alge, Daniel

(57) **Zusammenfassung**

Die Erfindung betrifft eine stabile pharmazeutische Präparation, die ein Protein mit Faktor VIII-Prokoagulationsaktivität und vWF-Bindungsaktivität enthält, wobei das Protein eine Aminosäuresequenz hat, die von der Aminosäuresequenz des Faktor VIII-Proteins abgeleitet ist und mindestens eine Mutation in mindestens einer immundominanten Region von Faktor VIII aufweist. Weiters sind Verfahren zur Herstellung einer solchen stabilen pharmazeutischen Präparation und medizinische Verwendungen für solche Präparationen beschrieben.

## Beschreibung

Die Erfindung betrifft eine stabile pharmazeutische Präparation mit Faktor-VIII-Prokoagulationsaktivität und vWF-Bindungsaktivität.

Faktor VIII fungiert auf dem intrinsischen Weg der Blutkoagulation als Cofaktor in Gegenwart von Ca²⁺ und Phospholipiden für die Faktor IXa-abhängige Umwandlung von Faktor X zu Xa. Die molekulare Klonierung von Faktor VIII-cDNA zeigte, daß Faktor VIII aus einer Reihe homologer Domänen besteht, die wie folgt dargestellt werden können: A1-A2-B-A3-C1-C2. Im Plasma zirkuliert Faktor VIII als ein an ein Metallion gebundenes Hetero-Dimer, welches nicht-kovalent an von Willebrand-Faktor (vWF) gebunden ist. Die schwere Kette von Faktor VIII mit einem Molekulargewicht von 90 000 bis 200 000 weist die A1 und die A2-Domänen sowie einen variablen Teil der B-Domäne auf. Die leichte Kette von Faktor VIII von 80 kDa besteht aus den Domänen A3, C1 und C2. Faktor VIII-Aktivierung erfolgt durch begrenzte Proteolyse durch Thrombin an den Aminosäurepositionen Arg³⁷², Arg⁷⁴⁰ in der schweren Kette und Arg¹⁶⁸⁹ in der leichten Kette. Folglich besteht der aktivierte Faktor VIII aus einem Hetero-Trimer der separaten Al- und A2-Domänen zusammen mit einer mit Thrombin geschnittenen leichten Kette.

Die mit dem Faktor X verbundene Bluterkrankheit Hämophilie A wurde mit dem (funktionellen) Fehlen von Faktor VIII in Verbindung gebracht. Im Laufe der Jahre wurde der molekulare Defekt im Faktor VIII-Gen bei einer beeindruckenden Anzahl von Hämophilie-A-Patienten gefunden (Tuddenham et al., NAR 22 (1994), 3511-3533; Antonarakis et al., Human Mutation 5 (1995), 1-22). Besonderes Interesse wurde auf Hämophilie-A-Patienten gerichtet, die in ihrem Plasma kreuzreagierendes Material (cross reacting material, CRM⁺) enthalten. Elegante Untersuchungen, bei welchen eine Mikroreinigung des Faktor VIII aus Plasma von CRM⁺-Hämophilie A-Patienten verwendet wurde, trugen wesentlich zum derzeitigen Wissen über die Struktur-Funktion-Beziehungen von Faktor VIII bei. Es zeigte sich, daß durch Mutationen an den Thrombin-Schnittstellen an den Aminosäurepositionen Arg³⁷² und Arg¹⁶⁸⁹ die Aktivierung von Faktor VIII beeinflußt werden, was mit Daten, die mit rekombinanten Proteinen erhalten wurden, in welchen die Schnittstellen von Thrombin durch stellengerichtete Mutagenese geändert worden sind, übereinstimmt.

Weiters zeigte es sich, daß zwei Mutationen im Faktor VIII-Gen zu einer zusätzlichen Glykosylierung des Proteins, das die Faktor VIII-Aktivität beeinflußte, führt. Die molekulare Basis des genetischen Defekts bei Patienten mit einem verringerten Faktor VIII-Spiegel in ihrem Plasma wurde weniger erforscht. Higuchi und Mitarbeiter identifizierten eine Mutation im Faktor VIII-Gen eines an einer leichten Hämophilie A leidenden Patienten, die zur Substitution eines Phenylalanins durch ein Tyrosin an der Aminosäureposition 1680 führte (PNAS 88 (1991), 8307-8311). Ein diese bestimmte Aminosäuresubstitution enthaltendes rekombinantes Faktor VIII-Protein erwies sich als mangelhaft bei der Bindung an vWF.

Eine andere Mutation des Faktor VIII-Moleküls, in welchem das Arginin an der Aminosäureposition 2307 durch ein Glutamin ersetzt ist, wurde mit leichter bis mäßiger Hämophilie A in Verbindung gebracht (Gitschier et al., Science 232 (1986), 1415-1416; Casula et al., Blood (1990), 662-670). Die funktionelle Bedeutung des Restes Arg²³⁰⁷ bei der Funktion von Faktor VIII wird durch die Fähigkeit eines diesen bestimmten Rest enthaltenden synthetischen Peptids, die Bindung von Faktor VIII an Phospholipide zu inhibieren, indiziert. Weiters wurden Belege dafür vorgelegt, daß dieser Teil des nativen Faktor VIII-Moleküls an vWF binden kann. Anfängliche Versuche, den Defekt, der durch diese Mutation bewirkt wird, unter Verwendung von aus Plasma gereinigtem Material zu charakterisieren, waren ohne Erfolg.

Anderseits ist es bekannt, daß etwa 20% der Hämophilie-A-Patienten, die mit Faktor VIII-Konzentraten behandelt werden, Faktor VIII-Inhibitoren (d.h. Antikörper gegen Faktor VIII) entwickeln, wodurch die Wirkungen der verabreichten Faktor VIII-Präparationen inhibiert werden.

Die Behandlung von Faktor VIII-Inhibitor-Patienten ist sehr schwierig, und bisher gibt es nur eine begrenzte Anzahl spezieller Verfahren zur Behandlung dieser Hämophilie A-Inhibitor-Patienten.

Es ist möglich, jedoch sehr teuer, hohe Dosen von Faktor VIII zu verabreichen und dadurch die Faktor VIII-Antikörper in vivo zu neutralisieren. Der überschüssige Faktor VIII wirkt dann hämostatisch. In vielen Fällen erfolgt eine Desensibilisierung, und es ist wiederum möglich, Faktor VIII-Standardbehandlungen anzuwenden. Eine solche Behandlung mit hoher Dosis erfordert jedoch große Mengen an Faktor VIII, ist zeitaufwendig und kann mit schweren anaphylaktischen Nebenreaktionen verbunden sein.

Eine andere kostenintensive Methode zur Entfernung von Faktor VIII-Inhibitoren arbeitet mit der extrakorporalen Immunadsorption an Lektinen, die an Immunoglobuline (Protein A, Protein G) oder an immobilisierten Faktor VIII binden. Da der Patient während dieser Behandlung mit der Apherese-Maschine verbunden sein muß, ist dieses Verfahren auch eine große Belastung in finanzieller Hinsicht und für den Patienten. Abgesehen davon ist es auch nicht möglich, mit diesem Verfahren die Blutgerinnung einer akuten Blutung zu erreichen.

Bisher war jedoch die Therapie der Wahl die Verabreichung von aktivierten Prothrombin-Komplex-Konzentraten (activated prothrombin complex concentrates, APCC), wie FEIBA® und AUTOPLEX®, welches selbst bei Patienten mit hohem Inhibitor-Titer zur Behandlung akuter Blutungen geeignet ist (vgl. z.B. DE-PS 31 27 318).

Andere Verfahren, die derzeit intensiv untersucht werden, sind die Verabreichung von Immunglobulin-Präparationen, die anti-idiotypische Antikörper enthalten, und die Verabreichung von rekombinantem Faktor VIIa. Doch für beide Verfahren ist es bisher nicht möglich, hinsichtlich ihrer klinischen Wirksamkeit eine endgültige Beurteilung zu treffen.

Es ist ein Ziel der vorliegenden Erfindung, eine pharmazeutische Präparation zur Behandlung von Patienten mit Faktor VIII-Inhibitoren und verwandten Blutgerinnungsstörungen zu schaffen, die eine einfache Verabreichung, eine hohe Stabilität des zu verabreichenden Produkts und eine wirksame Behandlung und eine lange Halbwertszeit ermöglicht und damit die Belastung der Patienten verringert.

Dieses Ziel wird durch eine stabile pharmazeutische Präparation gelöst, die ein Protein mit Faktor VIII-Prokoagulationsaktivität und vWF-Bindungsaktivität enthält, welches Protein eine Aminosäuresequenz hat, die von der Aminosäuresequenz des Faktor VIII-Proteins, vorzugsweise des Human-Faktor VIII-Proteins abgeleitet ist, mit mindestens einer Mutation in mindestens einer immundominanten Region von Faktor VIII.

Ein immundominanter Bereich des Faktor VIII-Proteins ist als Epitop, Struktur oder Domäne des Proteins, welche(s) primär eine Antikörperbildung bewirkt, definiert.

Die Mutation(en) kann (können) eine Punktmutation sein, welche zum Ersatz einer Aminosäure durch eine andere führt, Substitutionen, Deletionen (z.B. Deletionen von Regionen, die für die Wirkung von Faktor VIII in vivo nicht wesentlich sind) oder Insertionen (z.B. Verdoppelung bestimmter Regionen) sein.

Vorzugsweise liegt (liegen) die Mutation(en) in der C2 und/oder der A2-Domäne des Faktor VIII-Moleküls vor.

Gemäß der vorliegenden Erfindung sollte das in der Präparation enthaltene Protein sowohl Faktor VIII-Prokoagulationsaktivität als auch vWF-Bindungsaktivität aufweisen.

Es ist vorteilhaft, daß das mutierte Faktor VIII-Protein in der pharmazeutischen Präparation eine Faktor VIII-Prokoagulations-aktivität und/oder vWF-Bindungsaktivität von mindestens 30%, vorzugsweise mindestens 50%, mehr bevorzugt mindestens 80%, insbesondere mindestens 100%, der Faktor VIII-Prokoagulations-aktivität und/oder der vWF-Bindungsaktivität eines Faktor VIII-Proteins ohne die Mutation in der immundominanten Region, beispielsweise einer im Handel erhältlichen Faktor VIII-Präparation auf Basis von rekombinantem Faktor VIII:C, aufweist.

Die Bewertung der Faktor VIII-Prokoagulationsaktivität und der vWF-Bindungsaktivität kann mittels jedes geeigneten Tests für diese Eigenschaften vorgenommen werden, insbesondere jener Tests, die routinemäßig bei der Untersuchung von Faktor VIII-Proben durchgeführt werden, wie der Einstufen-Gerinnungstest, ein chromogener Test, wie Faktor VIII IMMUNOCHROM (IMMUNO) und die Adsorption des Faktor VIII an immobilisiertem vWF bzw. umgekehrt (vgl. auch Veltkamp et al., Thromb. Diäth. Haemos. 19 (1968), 279-303).

Die Faktor VIII-Koagulationsaktivität des mutierten Proteins gemäß der vorliegenden Erfindung wird vorzugsweise mittels eines "Einstufen-Gerinnungstests", wie z.B. in Mikaelsson und Oswaldsson, Scand. J. Haematol. Suppl. 33 (1984), 79-86, beschrieben, getestet.

Die Faktor VIII-Aktivität kann auch durch Messen der Fähigkeit von Faktor VIII, als Cofaktor für Faktor IXa bei der Umwandlung von Faktor X zu Faktor Xa unter Verwendung eines chromogenen Substrats für Faktor Xa (Coatest Factor VIII, Chromogenix, Moelndal, Schweden) zu fungieren, beurteilt werden. Außerdem können andere Tests, die zur Bestimmung der Menge an Faktor VIII-Aktivität in einer Probe dienen, zum Testen der Faktor VIII-Aktivität der mutierten Proteine, die in der vorliegenden Erfindung beschrieben sind, verwendet werden.

Der tatsächliche Test, ob irgendein neues, mutiertes Faktor VIII-Protein einen bestimmten Prozentanteil an Faktor VIII-Prokoagulationsaktivität aufweist, erfolgt vorzugsweise parallel zu einem Test auf dasselbe Faktor VIII-Molekül ohne die Mutation in der immundominanten Region (z.B. Faktor VIII-Wildtyp oder ein voll aktiver Faktor VIII mit deletierter B-Domäne). Mit einem derart geeichten Test des mutierten Faktor VIII-Moleküls kann die relative Prokoagulationsaktivität (der Prozentanteil der Aktivität im Vergleich zu 100% Aktivität des Wildtyps oder des Faktor VIII mit deletierter B-Domäne) ohne das Risiko eines methodenbedingten Fehlers getestet werden.

Die vWF-Bindungsaktivität kann auch mittels jedes Testsystems, mit dem ein Faktor VIII:C/vWF-Komplex bestimmt werden kann, der sich im Verlauf des Tests auf vWF-Bindung gebildet hat, getestet werden. Die Bindung des mutierten Faktor VIII-Proteins an vWF kann unter Verwendung von in der Literatur beschriebenen Tests (z.B. Leyte et al., Biochem. J., 257 (1990), S. 679-683; Donath et al., Biochem. J. 312 (1995), S. 49-55) vorgenommen werden. Bei diesen Tests kann gereinigter oder nicht gereinigter vWF verwendet werden. Der verwendete vWF ist vorzugsweise gereinigtes vWF-Protein. Diese Verfahren inkludieren die Beschichtung von gereinigtem vWF auf Mikrotiter-Vertiefungen (Leyte et al., Biochem. J., 257 (1990), S. 679-683), sind jedoch nicht auf diese beschränkt. Alternativ kann gereinigter oder nicht-gereinigter vWF mit Hilfe monoklonaler Antikörper, die gegen vWF gerichtet sind, immobilisiert (Leyte et al., Biochem. J., 274 (1991), S. 257-261; Donath et al., Biochem. J., 312 (1995), S. 49-55). Danach werden verschiedene Mengen an Faktor VIII zum immobilisierten vWF zugegeben, und die Menge an gebundenem Faktor VIII wird mittels üblicher Methoden bestimmt. Der oben beschriebene Test kann verwendet werden, um die Bindung von mutierten Faktor VIII-Proteinen an vWF zu beurteilen. Auf ähnliche Weise können andere Verfahren, die zur Bestimmung der Affinität eines Faktor VIII-Proteins zu vWF dienen, verwendet werden, um die vWF-Bindungseigenschaften der in der vorliegende Erfindung geoffenbarten, mutierten Proteine zu beurteilen.

Da bei in vitro-Tests auf Faktor VIII-Prokoagulationsaktivität und vWF-Bindungsaktivität die Ergebnisse oft durch auf ihr künstliches Design zurückzuführende Fehler beeinträchtigt sein können, werden beide Eigenschaften vorzugsweise auch mittels in vivo- oder ex vivo-Tests getestet, um verläßlichere Ergebnisse in bezug auf die Aktivitätswerte zu erhalten.

Wie bei den in vitro-Tests ist ein paralleles Testen des Faktor VIII-Moleküls ohne die Mutation in der immundominanten Region auch bevorzugt, wenn man die in vivo-Tests durchführt. Geeignete Tiermodelle zur Bewertung der Faktor VIII:C-Aktivität in Gegenwart eines Inhibitors sind in der WO 95/01570 und der EP 0 747 060 A beschrieben.

Gemäß einer bevorzugten Ausführungsform zeigt das mutierte Faktor VIII-Protein in der stabilen pharmazeutischen Präparation der vorliegenden Erfindung die Faktor VIII-Prokoagulationsaktivität und/oder die vWF-Bindungsaktivität in Gegenwart eines Faktor VIII-Inhibitors, der aus Inhibitor-Patienten isoliert werden kann, oder eines mittels Hybridom-Technologie erzeugten äquivalenten Antikörpers, insbesondere in Gegenwart von anti-Faktor VIII-Antikörpern, einschließlich doch nicht ausschließlich Antikörper menschlichen Ursprungs, die gegen die C2-Domäne und die A2-Domäne von Faktor VIII gerichtet sind, monoklonale Antikörper der Maus, einschließlich doch nicht ausschließlich CLB-CAg 117. Es ist bevorzugt, eine Faktor VIII-Mutante zu schaffen, die in Gegenwart einer Vielzahl von Inhibitoren aktiv ist, zumindest in Gegenwart von zwei Antikörpern, die an die immundominante Region des Faktor VIII-Moleküls binden.

Die Tatsache, daß diese bevorzugten mutierten Proteine durch Faktor VIII-Inhibitoren nicht inhibiert oder nur in geringem Ausmaß inhibiert sind, macht sie für die erfolgreiche Behandlung von Faktor VIII-Inhibitor-Patienten geeignet.

Bevorzugte Mutationen des Faktor VIII-Moleküls in der erfindungsgemäßen Präparation sind jene in immundominanten Epitopen oder Regionen von Faktor VIII, insbesondere in der C2- oder A2-Domäne. Bevorzugte Regionen für stellengerichtete Mutagenese sind um Arg²³⁰⁷ und Arg⁵⁹³, beispielsweise in der Region der Aminosäuren 2182 bis 2332, wie Thr²³⁰³-Trp²³¹³. Arginin ist dann vorzugsweise zu Glutamin oder Cystein substituiert. Bevorzugte Beispiele sind die Mutationen R2307Q (Arginin an der Stelle 2307 wird durch Glutamin ersetzt) und R593C (Arginin an der Stelle 593 wird durch Cystein ersetzt). Eine weitere immundominante Region der A2-Domäne befindet sich zwischen Arg⁴⁸⁴ und Ile⁵⁰⁸ (Healey G.F. et al., JBC 270 (1995), 14505-14509), und die Regionen um diese Stelle herum. Jede neue Mutation kann, wie oben angegeben, getestet werden, ob sie die für die erfindungsgemäße Präparation notwendigen Eigenschaften aufweist.

Die Faktor VIII-Mutante gemäß der vorliegenden Erfindung weist vorzugsweise eine weitere Mutation auf, nämlich eine Deletion mindestens eines Teils der B-Domäne, einer Region, die für die physiologische Aktivität von Faktor VIII nicht wesentlich ist. Deletionsmutanten, wie in EP-0 690 126 beispielhaft angeführt, können zur Erzeugung der Mutation gemäß der vorliegenden Erfindung verwendet werden. Die Vergleichstests für Faktor VIII-Koagulationsaktivität und für vWF-Bindungsaktivität für eine solche erfindungsgemäße Faktor VIII-Mutante mit deletierter B-Domäne werden dann vorzugsweise parallel mit einem Faktor VIII-Protein mit deletierter B-Domäne ohne die Mutation in der immundominanten Region durchgeführt.

Weitere Mutanten und/oder Fragmente sowie Derivate des Faktor VIII-Proteins können verwendet werden, solange sie eine Faktor VIII:C-Aktivität und eine vWF-Bindungsaktivität aufweisen.

Es zeigte sich überraschenderweise, daß die Faktor VIII-Mutante gemäß der Erfindung die vWF-Bindungsaktivität noch immer beibehielt, obwohl sie nicht durch übliche Inhibitoren inhibiert ist. Diese Feststellung ist umso überraschender, weil es auf dem Gebiet bekannt war, daß die Bindungsstellen für vWF und Inhibitoren einander überlappen.

Die Bindung an vWF in vitro oder in vivo ist eine Voraussetzung für die Stabilität der Faktor VIII-Mutante in einer Präparation oder nach Verabreichung der Präparation an den Patienten. In Gegenwart von vWF wird die Faktor VIII:C-Aktivität stabilisiert, so daß seine Aktivität selbst nach einer längeren Lagerzeit im wesentlichen erhalten bleibt. Auch der Faktor VIII:C-Spiegel in einem Patienten ist selbst nach einer längeren Zeitdauer nach der Verabreichung infolge der vWF-Bindung und einer längeren Halbwertszeit der so stabilisierten Faktor VIII-Mutante erhöht.

Ein weiterer Aspekt der vorliegenden Erfindung betrifft einen biologisch aktiven Faktor VIII-Komplex, welcher eine Faktor VIII-Mutante, wie oben beschrieben, und vWF oder ein vWF-Fragment aufweist. Es ist bevorzugt, rekombinanten vWF zu verwenden, wie in EP-0 197 592 beschrieben.

Noch ein anderer Aspekt der vorliegenden Erfindung ist ein Verfahren zur Herstellung einer pharmazeutischen Präparation gemäß der vorliegenden Erfindung, welches die Expression eines mutierten Faktor VIII-Proteins, wie oben beschrieben, in Säugetier-Zellen, die Reinigung dieses mutierten Proteins von diesen Zellen und die Formulierung des gereinigten mutierten Proteins zu einer pharmazeutischen Präparation aufweist.

Die Expressions- und Reinigungsschritte können gemäß irgendeinem bekannten Verfahren für die rekombinante Erzeugung von Faktor VIII, Faktor VIII-Mutanten oder verwandten Proteinen durchgeführt werden. Die Formulierung zu einer pharmazeutischen Präparation kann die Zugabe von pharmazeutisch akzeptablen Zusätzen, Stabilisatoren, weiteren aktiven Komponenten, sowie die Schaffung einer geeigneten Dosierungsform und einer gebrauchsfertigen Packung umfassen.

Die Expression erfolgt vorzugsweise bei Temperaturen im Bereich von 20 bis 45°C. Zur Erzeugung von in dieser Erfindung geoffenbarten mutierten Proteinen kann sich eine Expression bei einer Temperatur von 25 bis 37°C als geeignet erweisen, um bedeutende Mengen an Faktor VIII im konditionierten Medium zu erhalten, mehr bevorzugt kann eine Temperatur von 28°C verwendet werden, um die Expression mutierter Proteine zu erleichtern.

Es ist auch möglich, das mutierte Faktor VIII-Protein zusammen mit vWF oder Fragmenten davon unter Verwendung von Standard-Techniken für die Coexpression zu co-exprimieren.

Alternativ ist es auch möglich, das mutierte Faktor VIII-Protein vom Plasma eines Patienten, der diese spezielle Mutation in einer immundominanten Region von Faktor VIII trägt, zu reinigen und das gereinigte Protein zur Herstellung einer stabilen pharmazeutischen Präparation gemäß der Erfindung zu verwenden.

Die Zugabe von vWF und/oder vWF-Fragmenten erfolgt vorzugsweise während der Reinigungs- oder Formulierungsschritte.

Die Erfindung betrifft auch die Verwendung eines mutierten Faktor VIII-Proteins, wie oben beschrieben, zur Herstellung einer pharmazeutischen Präparation zur Behandlung von Patienten mit Faktor VIII-Inhibitoren oder Patienten mit einem Risiko für Faktor VIII-Inhibitoren.

Erfindungsgemäß werden solche Patienten mit einer wirksamen Dosis der vorliegenden stabilen pharmazeutischen Präparation behandelt. Die wirksame Dosis kann für jeden einzelnen Patienten gemäß der Schwere der Krankheiten, an der er bzw. sie leiden, und gemäß den Inhibitor-Titern des Patienten leicht bestimmt werden; die Dosierungen der Standard-Faktor VIII-Therapien können ein wichtiger Richtwert sein, auf den man als erste Dosierung der pharmazeutischen Präparation gemäß der vorliegenden Erfindung zurückgreift. Es ist wahrscheinlich (wenn auch nicht unbedingt notwendig), daß man die Dosierung während der weiteren Behandlung mit der vorliegenden Präparation infolge der längeren Halbwertszeit des mutierten Proteins verringern wird.

Gemäß einem weiteren Aspekt wird von der vorliegenden Erfindung eine Antikörper-Präparation vorgesehen, die Antikörper gegen ein mutiertes Faktor VIII-Protein, wie oben beschrieben, aufweist. Diese Antikörper-Präparation kann beispielsweise zur Reinigung oder zum Nachweis und/oder zur Bestimmung eines mutierten Faktor VIII-Proteins gemäß der vorliegenden Erfindung oder zur Unterscheidung zwischen der Faktor VIII-Mutante und nativem Faktor VIII, insbesondere im Blut oder Plasma des Patienten, verwendet werden.

Die Erfindung wird mittels der nachfolgenden Beispiele und der zugehörigen Zeichnungsfiguren genauer beschrieben, soll jedoch nicht auf diese beschränkt sein.

Es sind gezeigt in:

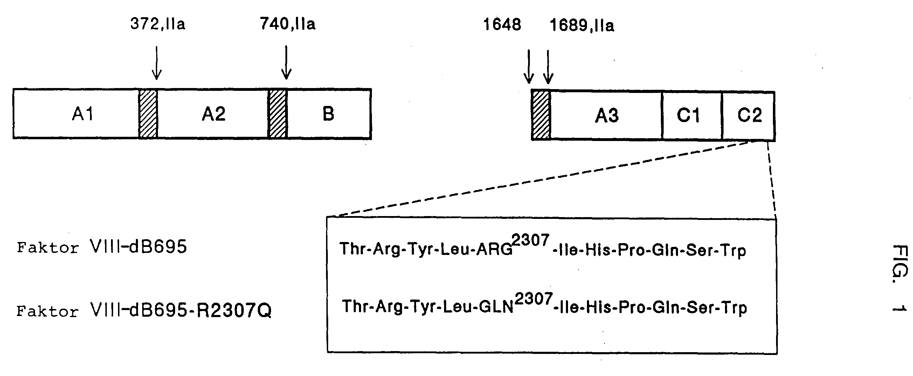

Fig. 1: eine schematische Darstellung von Faktor VIII dB695 und Faktor VIII dB695-R2307Q;
Fig. 2: die Pulse-Chase-Analyse von mit Faktor VIII dB695-cDNA transfektierten Zellen;
Fig. 3: die Pulse-Chase-Analyse von mit Faktor VIII dB695-R2307Q-cDNA transfektierten Zellen;
Fig. 4: Endoglycosidase H-Verdaue von Faktor VIII dB695 und Faktor VIII dB695-R2307Q;
Fig. 5: die Expression von Faktor VIII dB695 in SKHEP-Zellen;
Fig. 6 und Fig. 7: die Expression von Faktor VIII dB695-R2307Q in SKHEP-Zellen;
Fig. 8 und Fig. 9: Inhibition von Faktor VIII durch CLB-CAgll7;
Fig. 10: Faktor VIII-vWF-Bindung.

### Beispiele:

### BEISPIEL 1: Plasmid-Konstruktionen

Das Plasmid pCLB-BPVdB695,, das für eine Faktor VIII-Mutante codiert, in welcher Ala⁸⁶⁷ mit Asp¹⁵⁸³ verschmolzen ist, wurde von Mertens et al. (Brit. J. Haematol. 85 (1993), 133-145) beschrieben. Das Plasmid pCLB-BPVdB695 wurde durch Einführung eines synthetischen Linkers (5' TCGACCTCCAGTTGAACATTTGTAGCAAGCCACCATGGAAATAGAGCT 3'), der einen Teil der 5'-nicht-translatierten Region der Faktor VIII-cDNA, gebunden an eine Konsens-Sequenz zur Initiierung der Translation vor dem Start-Codon (unterstrichen) am 5'-Ende der Faktor VIII-cDNA enthält (Kozak, J. Biol. Chem. 266 (1991), 19867-19870), modifiziert. Am 3'-Ende, der PstI-Stelle an der Nucleotid-Position 7066 der Faktor VIII-cDNA (wobei Nucleotid 1 dem ersten Nucleotid des Start-Codons entspricht), wurde ein Linker inseriert, der eine NotI-Stelle enthält, die verwendet wurde, um die Faktor VIII dB695-cDNA in das Plasmid pBPV zu klonieren, was das Plasmid pCLB-dB695 ergibt. Das Plasmid pCLB-dB695-R2307Q wurde durch stellengerichtete Mutagenese unter Verwendung einer Überlappungsverlängerung (Ho et al., Gene 77 (1989) 51-59) konstruiert. Oligonucleotid-Primer RQ2307-1 (5' CGCTACCTTCAAATTCACCCC 3'; Nucleotid-Position 6967-6987 von Faktor VIII; Sense) und Oligonucleotid-Primer RQ2307-2 (5' CCATAGGTTGGAATCTAA 3'; Nucleotid 1221-1239 von pBPV; Anti-sense) wurden verwendet, um ein 302 bp-Fragment zu amplifizieren, das einen Teil der Faktor VIII-cDNA und das Plasmid pBPV enthielt. Der Oligonucleotid-Primer RQ2307-3 (5' TTAGGATCCCACTAAAGATGAGTTT 3'; Nucleotid-Position 5530-5547; Sense) wurde zusammen mit dem Oligonucleotid-Primer RQ2307-4 (5' GGGGTGAATTTGAAGGTAGCG 3'; Nucleotid-Position 6967-6987 von Faktor VIII; Anti-sense) verwendet, um ein 1464 bp-Fragment von Faktor VIII zu amplifizieren. Die Reaktionsbedingungen waren: 2 min 90°C, 20 min 50°C, 3 min 72°C; 37 mal 45 sec 90°C, 90 sec 50°C, 3 min 72°C; 5 min 65°C in Gegenwart von 1 mM dNTPs, Pfu-Polymerase-Reaktionspuffer, 50 pM jedes Oligonucleotid-Primers und 2,5 E Pfu-Polymerase (Lundberg et al., Gene 108 (1991), 1-6). Sowohl das 302 bp als auch das 1464 bp-Fragment wurden gereinigt und als Template in einer zweiten PCR unter Verwendung der Primer RQ2307-1 und RQ2307-4 verwendet, um ein 1738 bp-Fragment zu amplifizieren, das den Carboxy-Terminus der Faktor VIII-cDNA enthält, worin Arg²³⁰⁷ durch ein Gln ersetzt worden ist. Das amplifizierte Fragment wurde mit SalI und ApaI verdaut, und das resultierende 879 bp-Fragment, welches eine Mutation an der Aminosäureposition Arg²³⁰⁷ enthält, wurde verwendet, um das korrespondierende ApaI-SalI-Fragment von pCLB-dB695 zu ersetzen. Das resultierende Konstrukt wurde mit pCLB-dB695-R2307Q bezeichnet. Die Nucleotid-Sequenz des für die Konstruktion von pCLB-dB695-R2307Q verwendeten SalI-ApaI-Fragments wurde verifiziert.

### BEISPIEL 2: Gewebskultur und Transfektion

C127-Zellen wurde in mit 10% fötalem Kälberserum, 100 E/ml Penicillin und 100 pg/ml Streptomycin ergänztem Iscove-Medium gehalten. Subkonfluente Einzelschichten von C127-Zellen wurden im wesentlichen wie in Donath et al. (Biochem. J. 312 (1995), 49-55) beschrieben, transfektiert. Die Gegenwart von Faktor VIII-Protein im Kulturmedium wurde durch Messung sowohl der Faktor VIII-Aktivität als auch des Faktor VIII-Antigens (Mertens et al. (1993)) überwacht. Die Faktor VIII-Cofaktor-Aktivität wurde durch die Fähigkeit von Faktor VIII, als Cofaktor für die Faktor IXa-abhängige Bildung von Faktor Xa geschätzt, wobei ein chromogenes Substrat für Faktor Xa (Coatest Factor VIII, Chromogenix, Mölndal, Schweden) verwendet wurde. Faktor VIII-Antigen wurde unter Verwendung monoklonaler Antikörper, die zuvor charakterisiert wurden (vgl. Lenting et al., J. Biol. Chem. 264 (1994), 7150-7155), bestimmt. Monoklonaler Antikörper CLB-CAg 12, der gegen die leichte Kette von Faktor VIII gerichtet war, wurde als feste Phase verwendet, während mit Peroxidase markierter monoklonaler Antikörper CLB-CAg 117, der ebenso gegen die leichte Kette gerichtet, zur Quantifizierung der Menge des gebundenen Faktors VIII verwendet wurde. Die Menge der schweren Kette von Faktor VIII wurde, wie folgt, bestimmt: monoklonaler Antikörper ESH-5 (2,5 µg/ml) wurde an Mikrotiter-Vertiefungen über Nacht bei 4°C in 50 mM NaHCO₃ (pH 9,5) immobilisiert. Die Vertiefungen wurden mit 50 mM Tris-HCl (pH 7,4), 100 mM NaCl, 0,1% (Vol./ Vol.) Tween-20 gewaschen und 1 h lang im selben Puffer, enthaltend 1% (Gew./Vol.) HSA blockiert. Die Faktor VIII-Proben wurden in Blockierungs-Puffer verdünnt und 2 h lang bei 37°C mit dem immobilisierten Antikörper inkubiert. Danach wurden die Vertiefungen gewaschen, und die Menge an gebundenem Faktor VIII mit schwerer Kette wurde unter Verwendung von mit Peroxidase markiertem, monoklonalem Antikörper CLB-CAg 9 bei einer Konzentration von 0,7 pg/Vertiefung bestimmt. Die Nachweisgrenze dieses besonderen ELISA ist 10 mE/ml. Normalplasma aus einem Pool von 40 Spendern wurde als Standard verwendet. Die Ergebnisse dieser Expressions-Experimente sind in Tabelle 1 dargestellt (Werte als mE/ml ausgedrückt):

**Tabelle 1:**

| | Faktor VIII dB695 | Faktor VIII dB695-R2307Q |
|---|---|---|
| Cofaktor-Aktivität | 181,4 ± 2,4 | 1,73 ± 0,06 |
| Antigen (leichte Kette) | 199 ± 4,4 | nicht nachweisbar |
| Antigen (schwere Kette) | 225 ± 39 | nicht nachweisbar |

Im Gegensatz zu Faktor VIII dB695 konnten nur begrenzte Mengen an Cofaktor-Aktivität im Medium von Zellen, die mit Faktor VIII dB695-R2307Q-cDNA transfektiert worden waren, nachgewiesen werden. Mit dem für Faktor VIII mit leichter Kette spezifischen ELISA konnte kein immunreaktives Material nachgewiesen werden. Da die Gegenwart der Arg²³⁰⁷ zu Gln-Mutation die Bindung der verwendeten monoklonalen Antikörper beeinflussen kann, wurde Faktor VIII-Antigen untersucht, wobei der monoklonale Antikörper CLB-CAg 9, der gegen eine saure Region am Carboxy-Terminus der A2-Domäne von Faktor VIII gerichtet ist, in Verbindung mit dem monoklonalen Antikörper ESH-5, der gegen die schwere Kette von Faktor VIII gerichtet ist (Griffin et al., Thromb. Haemostasis 55 (1986), 40-46) verwendet wurde. Im Medium der Zellen, die mit Faktor VIII dB695-R2307Q-cDNA stabil transfektiert worden waren, konnte kein Faktor VIII-Antigen nachgewiesen werden, während Faktor VIII dB695 unter Verwendung dieser besonderen Antikörper-Kombination nachgewiesen werden konnte.

### BEISPIEL 3: metabolische Markierung und Immunpräzipitation

Transfektierte Zellen, die in mit 10% fötalem Kälberserum und 100 E/ml Penicillin und 100 pg/ml Streptomycin ergänztem Iscove-Medium gehalten wurden, wurden bei 80% Konfluenz einer metabolischen Markierung unterzogen. Die Zellen wurden zweimal mit PBS gewaschen und 30 min in RPMI (Voorberg et al., J. Cell. Biol. 113 (1991), 195-205) ohne Methionin, das mit 10% fötalem Kälberserum, gegen 25 mM HEPES (pH 7,0) dialysiert, ergänzt war, inkubiert. Danach wurden die Zellen 30 min lang in Anwesenheit von [³⁵S]-Methionin (50 µCi/ml, spez.Akt. >800 Ci/mmol) markiert. Medium von mittels Stoffwechsel markierten Zellen wurde in einem gleichen Volumen von zweifach konzentriertem Immunpräzipitationspuffer (immunoprecipitation buffer, IPB), bestehend aus 1% NP-40, 50 mM Tris-HCl (pH 7,5), 150 mM NaCl, 0,5% SDS, 10 µg/ml Sojabohnen-Trypsin-Inhibitor, 10 mM Benzamidin und 5 mM N-Ethylmaleimid, gesammelt. Die Zellen wurden zweimal mit Phosphat-gepufferter Kochsalzlösung (phosphate buffered saline, PBS) gewaschen, und die Zellen wurden in IPB lysiert. Die Lysate und die konditionierten Medien wurden entweder bei -20°C gelagert oder sofort zur Immunfällung verwendet. Die Zellextrakte und die konditionierten Medien wurden durch einstündige Inkubation bei Raumtemperatur mit Gelatine-Sepharose und zwei aufeinanderfolgende Inkubationen mit Protein A-Sepharose einem "Preclearing" unterzogen. Die spezifische Adsorption erfolgte über Nacht bei 4°C, durch vorgeformte Komplexe von Protein A-Sepharose mit polyklonalem Kaninchen-anti-Faktor VIII-Antiserum gegen aus Plasma gereinigten Faktor VIII. Die Immunpräzipitate wurden gründlich mit IPB und schließlich mit 20 mM Tris-HCl (pH 7,4) gewaschen und unter reduzierenden Bedingungen auf einem 7,5% (Gew./Vol.) SDS-Polyacrylamid-Gel analysiert. Nach der Elektrophorese wurden die Gele in 30% Methanol, 10% Essigsäure, fixiert und mit 20% Diphenyloxazol in Essigsäure 30 min lang behandelt. Danach wurden die Gele in H₂O inkubiert, getrocknet und verschiedene Zeiten lang belichtet. Endoglycosidase H-Verdaue von immunpräzipitiertem Material erfolgten gemäß Voorberg et al. (J. Cell Biol. 113(1991), 195-205).

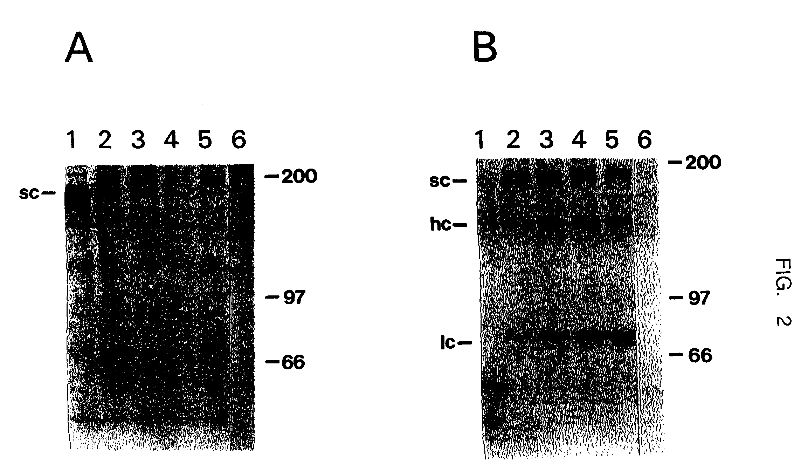

Mit den vorliegenden Versuchen konnte gezeigt werden, daß in der Zelle Faktor VIII dB695 als einzelkettiges Molekül auftritt, das im Lauf der Zeit allmählich aus der Zelle verschwindet (Fig. 2A: Analyse von Zellextrakten transfektierter Zellen bei verschiedeer Zeitdauer des "Chase" (der Zeitraum der Inkubation des Mediums mit den Zellen): Bahn 1: 0 Stunden "Chase", Bahn 2: 1 Stunde "Chase", Bahn 3: 3 Stunden "Chase" Bahn 4: 4 Stunden "Chase", Bahn 5: 6 Stunden "Chase", Bahn 6: Kontrolle; Fig. 2B: Analyse von konditioniertem Medium bei verschiedener Zeitdauer des "Chase": Bahn 1: 0 Stunden "Chase", Bahn 2: 1 Stunde "Chase", Bahn 3: 3 Stunden "Chase", Bahn 4: 4 Stunden "Chase", Bahn 5: 6 Stunden "Chase", Bahn 6: Kontrolle; Einzelkette (single chain, sc), leichte Kette (light chain, lc) und schwere Kette (heavy chain, hc) von Faktor VIII sind in der Figur angezeigt; Molekulargewicht-Marker sind an der rechten Seige der Figur angegeben).

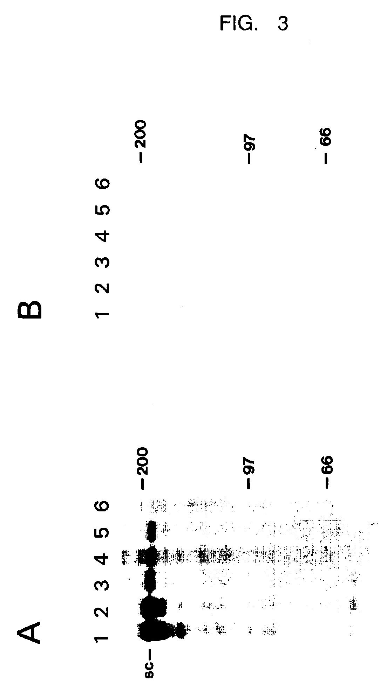

Gleichzeitig mit der Abnahme des in der Zelle beobachteten Signals wird eine Zunahme an Faktor VIII-reaktivem Material im Medium beobachtet. In Übereinstimmung mit früheren Daten wird Faktor VIII dB695 teilweise als einzelkettiges Protein ausgeschieden, während zwei Formen nachgewiesen werden können, die zuvor als leichte Kette und als schwere Kette, enthaltend einen Teil der B-Domäne von Faktor VIII dB695 charakterisiert worden sind (Mertens et al.(1993)). Die Biosynthese von Faktor VIII dB695-R2307Q innerhalb der Zelle ist ähnlich jener, die für Faktor VIII dB695 beobachtet wurde. Es konnte jedoch kein immunreaktives Material im Medium der stabil transfektierten Zellen nachgewiesen werden, was darauf hinweist, daß Faktor VIII dB695-R2307Q von der Zelle nicht sekretiert wird (Fig. 3A: Analyse von Zellextrakten transfektierter Zellen zu verschiedenen Zeiten des "Chase": Bahn 1: 0 Stunden, Bahn 2: 1 Stunde, Bahn 3: 3 Stunden, Bahn 4: 4 Stunden, Bahn 5: 6 Stunden, Bahn 6: Kontrolle; Fig. 3B: Analyse von konditioniertem Medium bei verschiedener Zeitdauer des "Chase": Bahn 1: 0 Stunden, Bahn 2: 1 Stunde, Bahn 3: 3 Stunden, Bahn 4: 4 Stunden, Bahn 5: 6 Stunden, Bahn 6: Kontrolle; Molekulargewicht-Marker sind rechts angegeben, und der Einzelketten-Faktor VIII dB695-R2307Q ist links in der Figur angegeben). Diese Beobachtung deutet an, daß der Großteil des anfangs synthetisierten Faktor VIII dB695-R2307Q innerhalb der Zelle über einen Mechanismus prozessiert wird, der ein anderer als der bei Faktor VIII dB695 ist.

### BEISPIEL 4: Biochemische Analyse von Faktor VIII innerhalb der Zelle

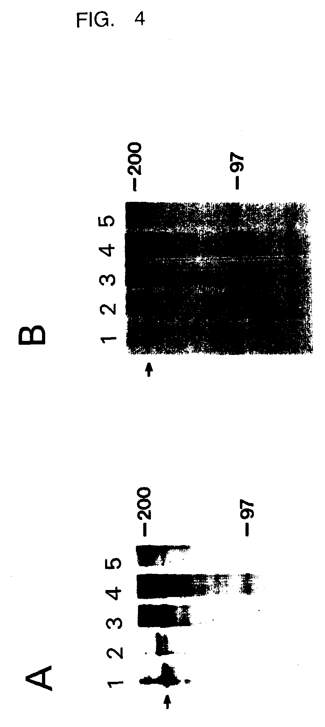

Um die intrazelluläre Lokalisierung von sowohl Faktor VIII dB695 als auch von Faktor VIII dB695-R2307Q zu beurteilen, wurde eine Pulse-Chase-Analyse durchgeführt. Die Sensibilität der immungereinigten Proteine gegenüber endo H schafft einen Marker für die intrazelluläre Lokalisierung beider Proteine. Wie in Fig. 4 gezeigt, bleiben sowohl Faktor VIII dB695 als auch Faktor VIII dB695-R2307Q gegenüber endo H zu den angegebenen verschiedenen Zeitpunkten empfindlich (Fig. 4A: mit Faktor VIII dB695-cDNA transfektierte Zellen: Bahn 1: 0 Stunden "Chase" + endo H, Bahn 2: 0 Stunden "Chase" - endo H, Bahn 3: 2 Stunden "Chase" + endo H, Bahn 4: 4 Stunden "Chase" + endo H, Bahn 5: 6 Stunden "Chase" + endo H; Fig. 4B: mit Faktor VIII dB695-R2307Q-cDNA transfektierte Zellen: Bahn 1: 0 Stunden "Chase" - endo H, Bahn 2: 0 Stunden "Chase" + endo H, Bahn 3: 2 Stunden "Chase" + endo H, Bahn 4: 4 Stunden "Chase" + endo H, Bahn 5: 6 Stunden "Chase" + endo H; Molekulargewichtmarker sind rechts von den Gelen angegeben.

Diese Ergebnisse deuten an, daß sowohl Faktor VIII dB695 als auch Faktor VIII dB695-R2307Q vorherrschend in einem Kompartiment vor dem medial-Golgi anwesend sind.

### BEISPIEL 5: Expression von Faktor VIII dB695 und Faktor VIII dB695-R2307Q bei 28°C und 37°C

Die in den vorherigen Abschnitten angeführten Ergebnisse zeigen an, daß Faktor VIII dB695-R2307Q aus Maus-Fibroblasten-C127-Zellen nur wenig sekretiert wird. Um zu beurteilen, ob die intrazelluläre Retention von Faktor VIII dB695-R2307Q ein für C127-Zellen spezifisches Phänomen ist, wurden Faktor VIII dB695-R2307Q- und Faktor VIII dB695-cDNA in SKHEP-Zellen exprimiert. Sowohl Faktor VIII dB695- als auch Faktor VIII dB695-R2307Q-cDNA wurde verwendet, um die Faktor VIII dB928-cDNA im Plasmid pCMV-dB928 (EP-0 711 835-A) zu ersetzen.

Das Plasmid pCLB-dB695 (vgl. Beispiel 1) wurde, wie folgt, modifiziert: Ein synthetischer, doppelsträngiger Linker 5' GGCCGCCCGGGC 3' wurde in die NotI-Stelle von pCLB-dB695 und pCLB-dB695-R2307Q inseriert. Danach wurde ein KpnI-XmaI-Fragment, das von pCLB-dB695 stammte und dem Carboxy-Terminus der Faktor VIII-cDNA entspricht, verwendet, um das korrespondierende KpnI-XmaI-Fragment von pCMV-dB928 zu ersetzen. Das resultierende Plasmid wurde als pCLB-CMV-dB695 bezeichnet. Auf ähnliche Weise wurde ein BglII-XmaI-Fragment, das dem Carboxy-Terminus der Faktor VIII-dB695-R2307Q-cDNA entspricht, verwendet, um das korrespondierende Fragment von pCMV-dB928 zu ersetzen, was zum Plasmid pCLB-CMV-dB695-R2307Q führte. SKHEP-Zellen wurden in mit 10% fötalem Kälberserum, 100 E/ml Penicillin und 100 µg/ml Streptomycin ergänztem Iscove-Medium gehalten. Subkonfluente Einzelschichten von SKHEP-Zellen wurden, im wesentlichen wie in Donath et al. (Biochem. J. 312 (1995), 49-55) beschrieben, transfektiert. Die transfektierten Zellen wurden bei Hygromycin-Konzentrationen von 100-1050 mg/ml selektiert, und einzelne Klone wurden isoliert und in selektivem Medium vermehrt. Die Anwesenheit von Faktor VIII-Protein im konditionierten Medium der transfektierten Zellen wurde durch Messung der Faktor VIII-Aktivität sowie des Faktor VIII-Antigens überwacht. Die Faktor VIII-Cofaktor-Aktivität wurde gemessen, indem die Fähigkeit von Faktor VIII, als Cofaktor für die Faktor IXa-abhängige Bildung von Faktor Xa zu fungieren, gemessen wurde, wobei ein chromogenes Substrat für Faktor Xa (Coatest Factor VIII, Chromogenix, Mölndal, Schweden) verwendet wurde. Faktor VIII-Antigen wurde unter Verwendung monoklonaler Antikörper, die zuvor charakterisiert wurden (vgl. Leyte et al., Biochem. J. 263 (1989) 187-194) bestimmt. Der gegen die leichte Kette von Faktor VIII gerichtete monoklonale Antikörper CLB-CAg 12 wurde als feste Phase verwendet, während mit Peroxidase konjugierter monoklonaler Antikörper CLB-CAg 69 oder CLB-CAg 117, der auch gegen die leichte Kette von Faktor VIII gerichtet war, zur Quantifizierung der Menge an gebundenem Faktor VIII verwendet wurde.

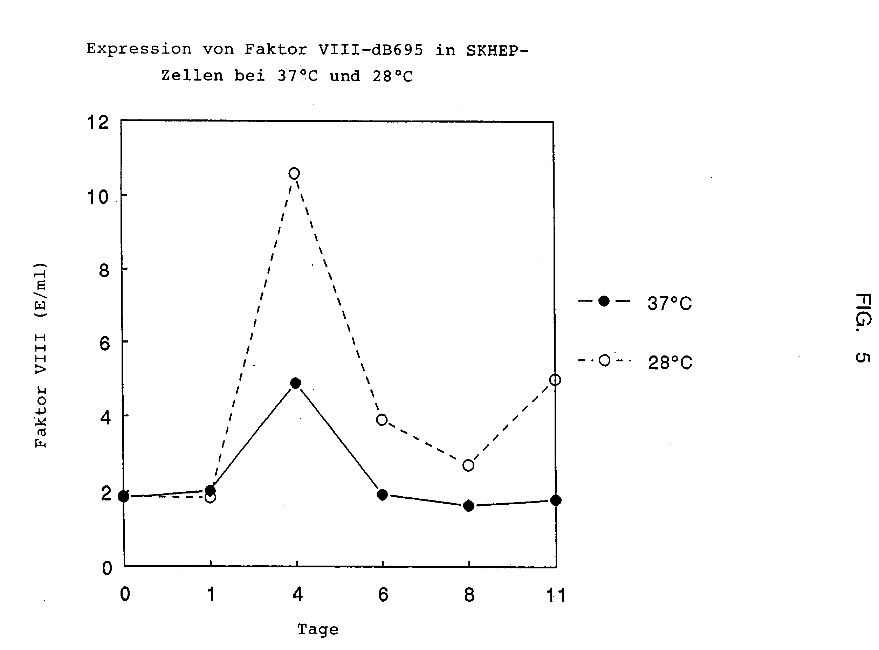

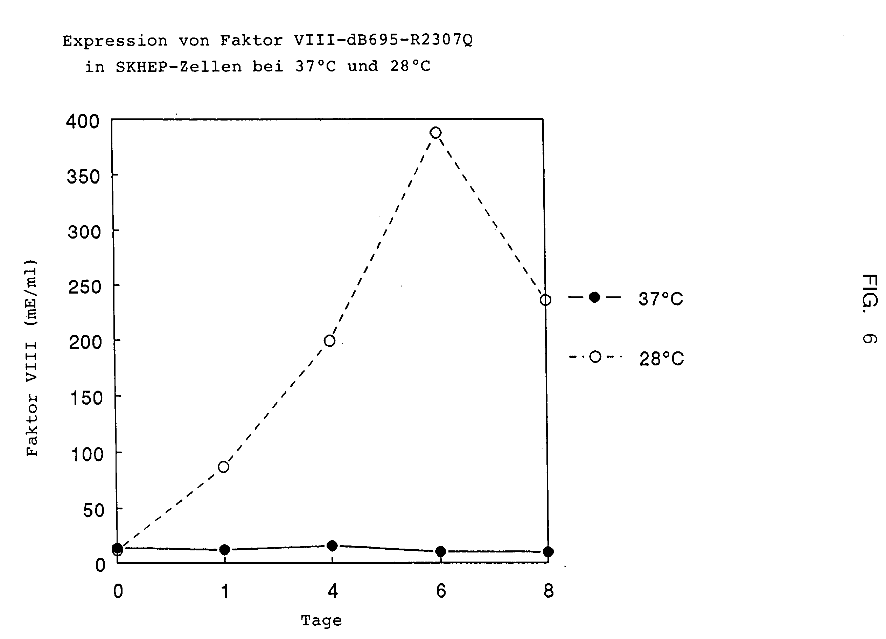

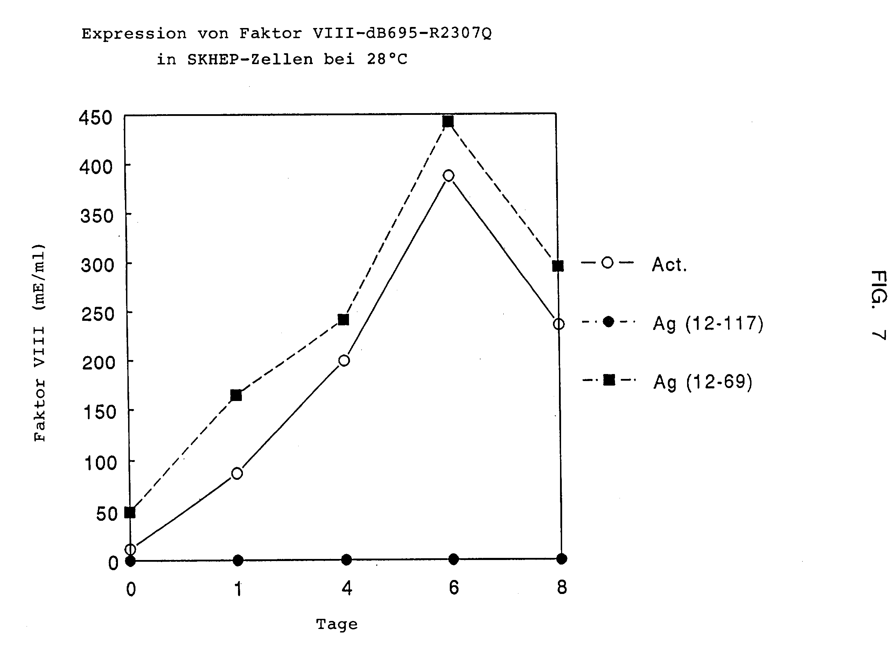

Transfektierte SKHEP-Zellen, die Faktor VIII dB695 exprimieren, wurden bei 28°C und 37°C gezüchtet, und die in das Medium sekretierte Menge an Faktor VIII-Protein wurde durch Bestimmung der Faktor VIII-Cofaktor-Aktivität, wie oben angegeben, überwacht. Bei 37°C wird Faktor VIII-dB695 von transfektierten SKHEP-Zellen mit einer Menge von 2 E/ml sekretiert. Am Tag 4 ist die Expression von Faktor VIII dB695 etwas höher und erreicht einen Wert von 5 E/ml (Fig. 5). Bei 28°C reicht die im konditionierten Medium angetroffene Menge an Faktor VIII-Aktivität von 2-10 E/ml (Fig. 5). Transfektierte SKHEP-Zellen, die Faktor VIII dB695-R2307Q exprimieren, wurden auf ähnliche Weise analysiert. Bei 37°C werden nur begrenzte Mengen an Faktor VIII-Aktivität aus der transfektierten Zelle sekretiert (± 10 mE/ml). Überraschenderweise trifft man bei 28°C bedeutende Mengen an Faktor VIII-Aktivität im konditionierten Medium von mit Faktor VIII dB695-R2307Q-cDNA transfektierten Zellen (Fig. 6) an. Danach wurde die Menge an Faktor VIII-Antigen im konditionierten Medium von SKHEP-Zellen, die stabil mit Faktor VIII dB695-R2307Q-cDNA transfektiert worden waren, bestimmt, wobei die oben beschriebenen monoklonalen Antikörper verwendet wurden. Die Menge an Faktor VIII-Antigen, wie sie mit den monoklonalen Antikörpern CLB-CAg 12 und CLB-CAg 69 bestimmt worden war, erwies sich als der Menge der im konditionierten Medium vorhandenen Cofaktor-Aktivität ähnlich (Fig. 7). Diese Beobachtung läßt erkennen, daß Faktor VIII dB695-R2307Q von transfektierten SKHEP-Zellen bei 28°C als funktionell voll aktives Faktor VIII-Protein sekretiert werden kann. Danach wurde die Menge an Faktor VIII-Antigen unter Verwendung der monoklonalen Antikörper CLB-CAg-12 und CLB-CAg 117 beurteilt. Überraschenderweise konnte kein Faktor VIII-Antigen im konditionierten Medium von mit Faktor VIII dB695-R2307Q-cDNA transfektierten Zellen unter Verwendung dieser bestimmten Kombination von monoklonalen Antikörpern nachgewiesen werden (Fig. 7). Diese Daten weisen darauf hin, daß der monoklonale Antikörper CLB-CAg 117 nicht mit Faktor VIII dB695-R2307Q reagiert. Eine Analyse transfektierter SKHEP-Zellen unter Verwendung der Immunfluoreszenz bestätigte, daß CLB-CAg 117 nicht mit Faktor VIII dB695-R2307Q reagierte. Die Beobachtungen eines Fehlens der Reaktivität von CLB-CAg 117 mit Faktor VIII dB695-R2307Q ließ erkennen, daß die Substitution der Aminosäure Arg²³⁰⁷→Gln in Faktor VIII die Bindung von monoklonalem Antikörper CLB-CAg 117 stört.

### BEISPIEL 6: Charakterisierung von monoklonalem Antikörper CLB-CAg 117

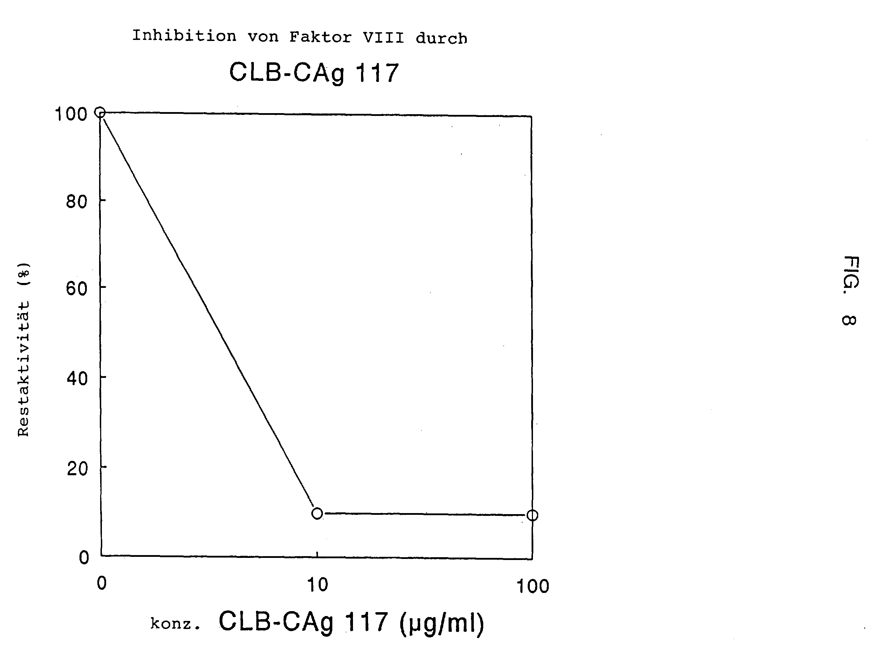

Im vorherigen Beispiel wurde gezeigt, daß der monoklonale Antikörper CLB-CAg 117 mit Faktor VIII dB695-R2307Q nicht reaktiv ist. Außerdem konnte gezeigt werden, daß Faktor VIII dB695-R2307Q ein funktionell voll aktives Faktor VIII-Protein ist, daß in SKHEP-Zellen bei 28°C exprimiert werden kann. Die Eigenschaften des monoklonalen Antikörpers CLB-CAg 117 wurden weiter charakterisiert. Das Epitop des monoklonalen Antikörpers CLB-CAg 117 wurde auf der leichten Kette von Faktor VIII lokalisiert. Die Beobachtung, daß der monoklonale Antikörper CLB-CAg 117 mit Faktor VIII dB695-R2307Q nicht reagiert, deutet sehr darauf hin, daß das Epitop dieses Antikörpers in der C2-Domäne von Faktor VIII lokalisiert ist. Die Plasmide pCLB-GP67-80K und pCLB-GP67-C2 wurden konstruiert. Das für die leichte Kette von Faktor VIII codierende Plasmid pCLB-GP67-80K wurde durch Amplifikation eines 689 bp-Fragments unter Verwendung der Oligonucleotid-Primer 80K-1 (5' GCCCCATGGGGGAAATAACTCGTACTACTC 3'; Nucleotid-Position 5000-5020 von Faktor VIII, Sense) und 80K-2 (5' CTGTACTGTCACTTGTCTCCC 3'; Nucleotid-Position 5659-5679 von Faktor VIII; Anti-sense) konstruiert. Das 689 bp-Produkt wurde gereinigt und mit NcoI und NdeI verdaut. Das Plasmid pCLB-dB695 wurde mit NdeI (Nucleotid-Position 5521 von Faktor VIII) und NotI verdaut, was zu einem Fragment führte, das dem Carboxyterminalen Teil der leichten Kette von Faktor VIII entspricht. Das NcoI-NdeI-Fragment und das NdeI-NotI-Fragment wurden zusammen in das Plasmid pAcGP67B (Pharmingen, San Diego, CA, USA) kloniert, und dies ergab das Plasmid pCLB-GP67-80K. Das Plasmid pCLB-GP67-C2 wurde unter Verwendung des Oligonucleotid-Primers C2-1 (5' GTGCCATGGGTAGTTGCAGCATGCCATTG 3'; Nucleotid-Position 6574-6591 von Faktor VIII; Sense) und des Primers C2-2 (5' CCATAGGTTGGAATGTAA 3'; Nucleotid-Position 1222-1239 von pBPV; Anti-sense), die zur Amplifikation eines der C2-Domäne von Faktor VIII entsprechenden Fragments verwendet wurden, konstruiert. Nach der Amplifikation wurde das Fragment mit NcoI und NotI verdaut und in das Plasmid AcGP67B kloniert. Die Nucleotidsequenz der klonierten Sequenz wurde durch Oligonucleotid-Sequenzierung verifiziert. Rekombinante Baculo-Viren, die die leichte Kette von Faktor VIII und die C2-Domäne von Faktor VIII exprimieren, wurden durch Transfektion in Sf-9-Zellen in Verbindung mit Baculogold™ Baculovirus Autographa californica DNA (Pharmingen, San Diego, CA, USA) erhalten. Rekombinante Viren, die die leichte Kette von Faktor VIII und die C2-Domäne von Faktor VIII exprimieren, wurden verwendet, um High Five™-Zellen mit einer Infektions-Multiplizität von 7 zu infizieren. Die Zellen wurden in einem Kulturmedium gehalten, das aus 25% (Vol./Vol.) Grace-Insektenmedium und 75% (Vol./Vol.) EX-CELL 401-Medium, mit 2,5% fötalem Kälberserum, 100 E/ml Penicillin und 100 mg/ml Streptomycin ergänzt, bestand. 24 h nach der Infektion wurden die Zellen mit [35S]Methionin (50 µCi/ml, spez. Akt. > 800 Ci/mmol) 24 Stunden lang in einem ähnlichen Kulturmedium, ohne Methionin, "Pulse"-markiert. Medium von mittels Stoffwechsel markierten Zellen wurde in einem gleichen Volumen von 2fach konzentriertem Immunpräzipitationspuffer (IPBB) gesammelt. IPBB besteht aus 50 mM Tris-HCl (pH 7,6); 1 M NaCl; 1,2% (Vol./Vol.) Triton-X-100; 0,1% (Gew./Vol.) Tween-20; 1,0% (Vol./Vol.) BSA; 35 mM EDTA; 10 µg/ml Sojabohnen-Trypsin-Inhibitor; 10 mM Benzamidin und 5 mM N-Ethylmaleimid. Immunfällung mit monoklonalem Antikörper CLB-CAg 117 wurde, wie folgt, durchgeführt. Konditioniertes Medium wurde durch zweistündige Inkubation mit Gelatin Sepharose 4B und zwei aufeinanderfolgende Inkubationen mit Protein G Sepharose 4FF bei Raumtemperatur einem "Preclearing" unterzogen. Die spezifische Adsorption erfolgte über Nacht bei 4°C durch Zugabe von 1 µg/ml des monoklonalen Antikörpers CLB-CAg 117 zu Protein G-Sepharose. Die Immunpräzipitate wurden gründlich mit IPBB und schließlich mit 20 mM Tris-HCl (pH 7,6) gewaschen. Das gebundene Protein wurde durch 5minütiges Kochen in SDS-PAGE-Probenpuffer eluiert und unter reduzierenden Bedingungen auf einem 10% (Gew./Vol.) SDS-Polyacrylamid-Gel analysiert. Nach der Elektrophorese wurden die Gele in 30% Methanol, 10% Essigsäure, fixiert und mit 10% Diphenyloxazol in Essigsäure 30 min lang behandelt. Schließlich wurden die Gele in H₂O inkubiert, getrocknet und verschiedene Zeit lang belichtet. Immunpräzipitation mit dem monoklonalen Antikörper CLB-CAg 117 zeigte, daß dieser besondere Antikörper sowohl mit der radioaktiv markierten leichten Kette des Faktors VIII als auch mit der C2-Domäne reagierte. Das Epitop von CLB-CAg 117 befindet sich in der C2-Domäne von Faktor VIII. Als solches ähnelt CLB-CAg 117 den anti-Faktor VIII-Antikörpern, die sich bei Hämophilie-A-Patienten nach Behandlung mit Faktor VIII bilden. Das Epitop eines signifikanten Anteils dieser anti-Faktor VIII-Antikörper befindet sich in der C2-Domäne. Ganz offensichtlich ergibt CLB-CAg 117 ein nützliches Modell zur Nachahmung der Wirkung der anti-Faktor VIII-Antikörper, die sich nach Behandlung mit Faktor VIII bei Patienten mit Hämophilie A bilden. Die Fähigkeit von CLB-CAg 117, Faktor VIII-Aktivität zu inhibieren, wurde kontrolliert. Verschiedene Mengen von CLB-CAg 117 wurden 2 h lang bei 37°C mit normalem Plasma inkubiert. Danach wurde die Faktor VIII-Aktivität des normalen Plasmas unter Verwendung eines einstufigen Gerinnungstests (Mertens et al., Brit. J. Haematol. 85 (1993), 133-145) bestimmt. Die Ergebnisse der Versuche sind in Fig. 8 gezeigt und zeigen, daß der monoklonale Antikörper CLB-CAg 117 ein starker Inhibitor der Faktor VIII-Aktivität ist. Schon 10 µg/ml CLB-CAg 117 genügen, um die Faktor VIII-Aktivität auf 10% ihres Anfangswertes zu verringern. Insgesamt zeigen diese Ergebnisse, daß CLB-CAg 117 sein Epitop an der C2-Domäne der leichten Kette von Faktor VIII hat und als starker Inhibitor der Faktor VIII-Aktivität wirkt. Die obigen Versuche zeigen, daß die Eigenschaften von CLB-CAg 117 jener von Human-allo- oder auto-Antikörpern, die gegen die C2-Domäne von Faktor VIII gerichtet sind, die sich entweder spontan oder als Folge einer Faktor VIII-Ersatz-Therapie von Patienten mit Hämophilie A entwickeln, stark ähneln.

### BEISPIEL 7: Inhibition von Faktor VIII dB695-R2307Q durch CLB-CAg 117

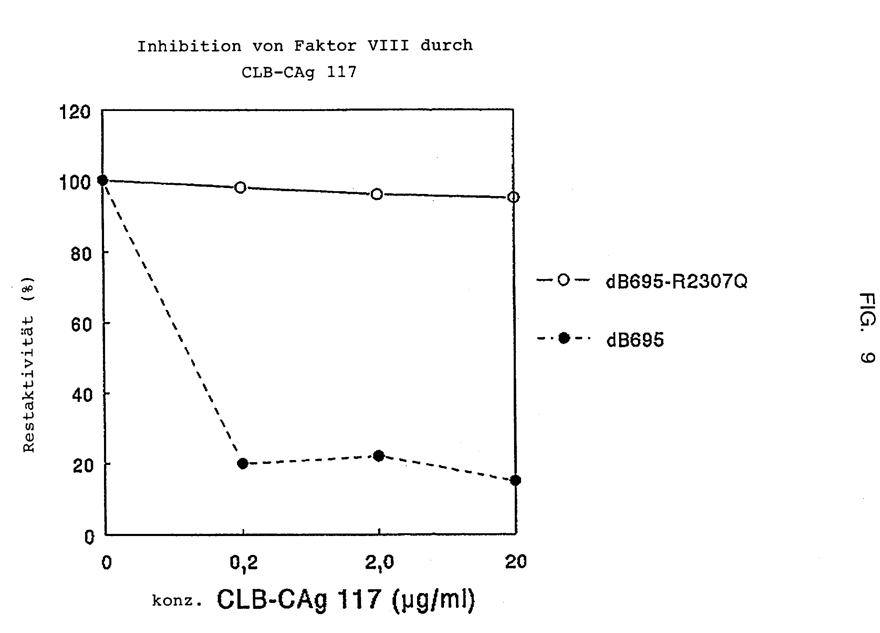

In den voranstehenden Beispielen wurde der monoklonale Antikörper CLB-CAg 117, ein inhibitorischer Antikörper, der gegen die C2-Domäne von Faktor VIII gerichtet ist, charakterisiert. In Beispiel 5 wurde die Expression von Faktor VIII dB695-R2307Q in SKHEP-Zellen beschrieben. Überraschenderweise zeigte es sich, daß die Sekretion von Faktor VIII dB695-R2307Q temperaturabhängig ist, und bedeutende Mengen an Faktor VIII dB695-R2307Q wurden bei 28°C in das konditionierte Medium sekretiert. Sekretierter Faktor VIII dB695-R2307Q erwies sich als funktionell aktiv. Eine Untersuchung der Reaktivität von CLB-CAg 117 unter Verwendung von sowohl einem Faktor VIII-Antigen-Test als auch Immunfluoreszenz zeigte, daß Faktor VIII dB695-R2307Q mit dem monoklonalen Antikörper CLB-CAg 117 nicht reagierte. Diese Beobachtung legt nahe, daß Faktor VIII dB695-R2307Q auch gegenüber Faktor VIII-inhibierender Aktivität von CLB-CAg 117 unempfindlich ist. Um diese Hypothese zu testen, wurde die Fähigkeit des monoklonalen Antikörpers CLB-CAg 117, Faktor VIII dB695-R2307Q zu inhibieren, bestimmt. Als Kontrolle wurde die Inhibition von Faktor VIII dB695 durch den monoklonalen Antikörper CLB-CAg 117 getestet. Verschiedene Mengen an CLB-CAg 117 wurden 2 h lang bei Raumtemperatur mit Faktor VIII dB695 oder Faktor VIII dB695-R2307Q inkubiert. Danach wurde die Faktor VIII-Restaktivität unter Verwendung eines einstufigen Gerinnungstests (Mertens et al., Brit. J. Haematol. 85 (1993), 133-145) bestimmt. Die Untersuchung des Musters, das für die Inhibition von Faktor VIII dB695 durch den monoklonalen Antikörper CLB-CAg 117 erhalten worden war, zeigte, daß schon eine geringe Menge von lediglich 0,2 µg/ml CLB-CAg 117 zu einer Restaktivität von 20% führte. Höhere Mengen von CLB-CAg 117 ergaben keine zusätzliche Inhibition der Faktor VIII-Aktivität. Das nach Inkubation von CLB-CAg 117 mit Faktor VIII dB695-R2307Q erhaltene Muster war ganz anders. Die Aktivität von Faktor VIII dB695-R2307Q konnte durch CLB-CAg 117 nicht inhibiert werden. Selbst eine große Menge an Antikörper (20 µg/ml) konnte die Faktor VIII-Aktivität von Faktor VIII dB695-R2307Q nicht blockieren (Fig. 9). Diese Ergebnisse zeigen, daß Punkt-Mutationen an immundominanten Bereichen von Faktor VIII verwendet werden können, um unter Beibehaltung der Faktor VIII-Aktivität die Bindung von inhibierenden Antikörpern selektiv zu behindern. Ähnlich der Substitution von Arg²³⁰⁷ durch Gln können andere Aminosäure-Substitutionen verwendet werden, um die Bindung von Faktor VIII-inhibierenden Antikörpern zu behindern.

### BEISPIEL 8: Bestimmung der vWF-Bindungsaktivität von Faktor VIII dB695-R2307Q

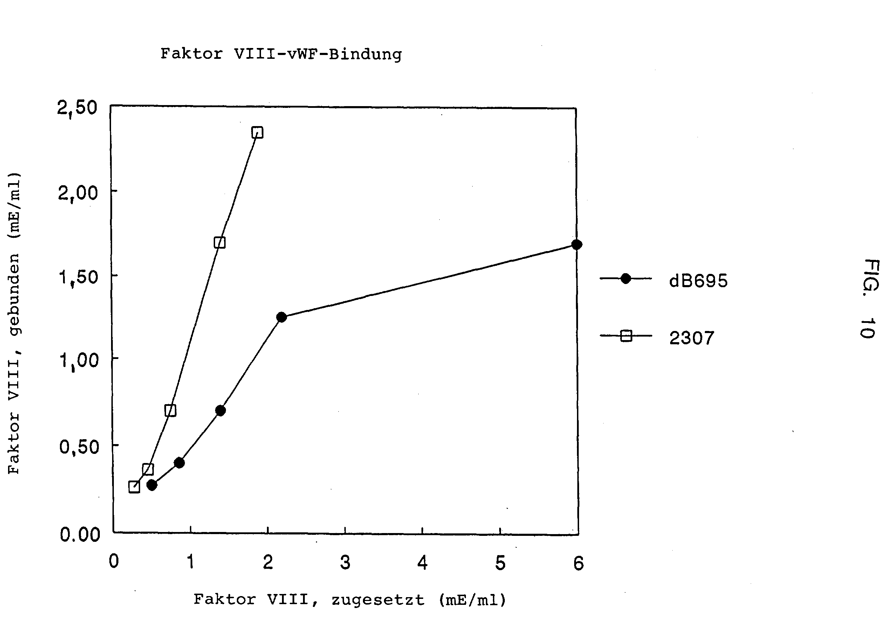

Im vorhergehenden Beispiel wurde gezeigt, daß CLB-CAg 117 die biologische Aktivität von Faktor VIII dB695-R2307Q nicht inhibieren kann. Die vWF-Bindungseigenschaften von Faktor VIII dB695-R2307Q wurden unter Verwendung eines bereits früher beschriebenen Bindungstests (Leyte et al., Biochem. J. (1990) Bd. 257, 679-683; Donath et al., Biochem. J. (1995) Bd. 312, 49-55) bestimmt. Gereinigter von Willebrand-Faktor (5 µg/ml) wurde an Mikrotiter-Vertiefungen über Nacht bei 4°C immobilisiert. Danach wurde serumfreies Medium, das verschiedene Mengen von Faktor VIII dB695-R2307Q und Faktor VIII dB695 enthielt, eine Stunde lang mit dem immobilisierten von Willebrand-Faktor inkubiert. Die Menge an Faktor VIII, die an den immobilisierten von Willebrand-Faktor bindet, wurde quantifiziert. Die Ergebnisse sind in Fig. 10 gezeigt, und aus diesen Daten geht hervor, daß Faktor VIII dB695 in Übereinstimmung mit früheren Daten (Mertens et al., Brit. J. Haematol., 85 (1993), 133-142) an immobilisierten von Willebrand-Faktor binden kann. Überraschenderweise wurde auch Faktor VIII dB695-R2307Q in quantitativer Weise an den immobilisierten von Willebrand-Faktor gebunden. Diese Ergebnisse deuten darauf hin, daß Faktor VIII dB695-R2307Q ähnlich wie Faktor VIII dB695 mit hoher Affinität an von Willebrand-Faktor binden kann. Die vorliegenden Daten zeigen, daß eine Arg²³⁰⁷→Gln-Substitution in Faktor VIII die von Willebrand-Faktor-Bindungseigenschaften des Moleküls nicht beeinflußt. Insgesamt zeigen diese Ergebnisse, daß Faktor VIII dB695-R2307Q normale Faktor VIII-Aktivität besitzt und an von Willebrand-Faktor binden kann. Weiters zeigte es sich, daß Faktor VIII dB695-R2307Q der inhibitorischen Wirkung eines anti-Faktor VIII-Antikörpers widersteht. Dies kann Faktor VIII dB695-R2307Q bei der Behandlung von anti-Faktor VIII-Antikörpern von Patienten, die die Koagulationsaktivität von Faktor VIII inhibieren können, geeignet machen. Folglich kann Faktor VIII dB695-R2307Q als Teil einer pharmazeutischen Präparation verwendet werden, die zur Behandlung von Patienten mit erworbener Hämophilie A oder zur Behandlung von Hämophilie A-Patienten, die allo-Antikörper nach einer Faktor VIII-Ersatz-Therapie entwickelt haben, verwendet wird.

### BEISPIEL 9: Präparation einer A2-Domänen-Mutante

In den vorhergehenden Beispielen wurde belegt, daß Faktor VIII dB695-R2307Q durch CLB-CAg 117, einen anti-Faktor VIII-Antikörper, der gegen die C2-Domäne von Faktor VIII gerichtet ist, nicht inhibiert werden kann. Anti-Faktor VIII-Antikörper können auch gegen andere Regionen des Faktor VIII-Proteins gerichtet sein. Ein immundominantes Epitop von Faktor VIII ist an der A2-Domäne von Faktor VIII vorhanden. Eine Gruppe von Patienten wurde auf das Auftreten von anti-Faktor VIII-Antikörpern, die gegen die A2-Domäne von Faktor VIII gerichtet sind, gescreent. Um das Screenen von Patienten zu erleichtern, wurden rekombinante Baculoviren, die die schwere Kette und die A2-Domäne von Faktor VIII exprimieren, konstruiert. Das für die A2-Domäne von Faktor VIII codierende Plasmid pCLB-GP67-A2, wurde durch Amplifikation eines 1135 bp-Fragments unter Verwendung der Oligonucleotid-Primer A2-1 (5' ATTCCATGGGATCAGTTGCCAAGAAGCAT 3'; Nucleotid-Position 1174-1191 von Faktor VIII, Sense) und A2-2 (5' CTTGCGGCCGCGGAGAATCATCTTGGTTCAATGGC 3'; Nucleotid-Position 2263-2277 von Faktor VIII, Anti-sense) konstruiert. Das 1135 bp-Fragment wurde gereinigt, mit NcoI und NotI verdaut und in das Plasmid pAcGP67B kloniert. Im resultierenden Konstrukt, das mit pCLB-GP67-A2 bezeichnet wurde, ist die Aminosäuresequenz Ser³⁷³-Arg⁷⁴⁰von Faktor VIII mit dem Leader-Peptid des sauren Glycoproteins GP67 verschmolzen. Das für die Aminosäure Ala¹-Arg⁷⁴⁰ der schweren Kette von Faktor VIII codierende Plasmid pCLB-GP67-90K wurde durch Amplifikation eines 548 bp-Fragments unter Verwendung der Oligonucleotid-Primer 90K-1 (5' TCTCCATGGGTGCCACCAGAAGATACTAC 3'; Nucleotid-Position 58-75 von Faktor VIII, Sense) und 90K-2 (5' ACATACTAGTAGGGCTCC 3'; Nucleotid-Position 577-594 von Faktor VIII; Anti-sense) konstruiert. Das 548 bp PCR-Produkt wurde gereinigt und mit NcoI und NdeI verdaut. Das Plasmid pCLB-BPVdB695 wurde mit NdeI (Nucleotid-Position 461 von Faktor VIII) und KpnI (Nucleotid-Position 1811 von Faktor VIII) verdaut, was zu einem 1351 bp-Fragment führte. Das Plasmid pCLB-GP67-A2 wurde mit KpnI und NotI verdaut, und das resultierende Fragment wurde zusammen mit dem NcoI-NdeI-Fragment und den KpnI-NotI-Fragmenten, die oben beschrieben wurden, in pAcGP67B kloniert. Das resultierende Konstrukt wurde mit pCLB-GP67-90K bezeichnet. Rekombinante Viren, die die schwere Kette von Faktor VIII und die A2-Domäne exprimieren, wurden, wie in Beispiel 5 beschrieben, hergestellt. Immunpräzipitationsversuche unter Verwendung von 20 µl Plasma von Patienten wurden, wie in Beispiel 5 beschrieben, durchgeführt. Die mittels Stoffwechsel markierte A2-Domäne und die schwere Kette von Faktor VIII wurden bei den nachstehend beschriebenen Untersuchungen verwendet. Screenen von Plasma einer Gruppe von Patienten mit Hämophilie A zeigte, daß Plasma, das von einem Patienten mit leichter Hämophilie A stammte, anti-Faktor VIII-Antikörper enthielt, die sowohl mit der mittels Stoffwechsel markierten A2-Domäne und der schweren Kette von Faktor VIII reaktiv waren. Die Entwicklung von anti-Faktor VIII-Antikörpern bei Patienten mit leichter Hämophilie ist relativ selten, da bedeutende Mengen an endogenem Faktor VIII oft im Plasma dieser Patienten vorhanden sind. Dies deutet darauf hin, daß die anti-Faktor VIII-Antikörper, die sich unter diesen Bedingungen entwickeln, gegen ein Epitop gerichtet sind, das auf exogenem Faktor VIII vorhanden ist und das nicht auf endogenem Faktor VIII lokalisiert ist. Zur Beurteilung dieser Möglichkeit, wurde die Mutation im Faktor VIII-Gen des Patienten mit dem anti-Faktor VIII-Antikörper durch Amplifikation der 26 Exons von Faktor VIII unter Verwendung der Polymerase-Kettenreaktion bestimmt. Das Vorhandensein von Punkt-Mutationen im Faktor VIII-Gen wurde unter Verwendung von Einzelstrang-Konformationspolymorphie (single stranded conformation polymorphism, SSCP) in Kombination mit einer direkten Sequenzierung der amplifizierten PCR-Fragmente untersucht. Nach SSCP wurde eine aberrante Migration eines PCR-Fragments, entsprechend dem Exon 12 des Faktor VIII-Gens, beobachtet. Eine direkte Sequenzierung offenbarte eine Punkt-Mutation (C→T), die einen Ersatz von Arg⁵⁹³ durch ein Cys voraussagte. Es ist wahrscheinlich, daß die Missense-Mutation eine Konformationsänderung im endogen synthetisierten Faktor VIII-Protein bewirkten könnte. Um diesem Punkt nachzugehen, wurde die Arg⁵⁹³→Cys-Mutation in das Konstrukt, das für die Faktor VIII-A2-Domäne codiert, eingeführt. Das für die A2-Domäne von Faktor VIII codierende Plasmid pCLB-GP67B-A2-R593C, bei welchem Arg⁵⁹³ durch ein Cys ersetzt ist, wurde durch stellengerichtete Mutagenese unter Verwendung einer Überlapp-Verlängerung konstruiert. Der Oligonucleotid-Primer R593C (5' GAGAATATACAATGCTTTCTCCC 3'; Nucleotid-Position 1822-1844 von Faktor VIII; Sense) wurde zusammen mit dem Oligonucleotid-Primer A2-2 zur Amplifikation eines Fragments von 476 bp verwendet. Der Oligonucleotid-Primer R593C-2 (5' GGGAGAAAGCATTGTATATTCTC 3'; Nucleotid-Position 1822-1844 von Faktor VIII; Anti-sense) und der Oligonucleotid-Primer A2-1 wurden zur Amplifikation eines 682 bp-Fragments verwendet. Beide amplifizierten Fragmente wurden gereinigt und als Template in einer PCR unter Verwendung von Oligonucleotid-Primer A2-1 und A2-2 benützt, um ein DNA-Fragment zu amplifizieren, welches gereinigt und danach mit NcoI und NotI verdaut und in das Plasmid pAcGP67B kloniert wurde, was pCLB-GP67-A2R593C ergab. Die Nucleotid-Sequenz der modifizierten A2-Domäne wurde durch Oligonucleotid-Sequenzierung verifiziert. Konditioniertes Medium, das die metabolisch markierte A2-Domäne mit der Arg⁵⁹³→Cys-Mutation enthielt, wurde, wie in den Beispielen 2 und 3 beschrieben, hergestellt. Immunpräzipitation der mittels Stoffwechsel markierten, modifizierten A2-Domäne, A2R593C bezeichnet, mit dem monoklonalen Antikörper CLB-CAg 9 zeigte, daß A2R593C im konditionierten Medium in Mengen vorhanden war, die der Wildtyp-A2-Domäne von Faktor VIII ähnlich waren. Der im Plasma des Patienten vorhandene Antikörper reagierte jedoch nicht mit der modifizierten A2-Domäne (A2R593C). Diese Beobachtung läßt darauf schließen, daß der anti-Faktor VIII-Antikörper, der sich im Patienten während der Behandlung mit Faktor VIII entwickelte, nur gegen Wildtyp-Faktor VIII gerichtet ist. Der anti-Faktor VIII-Antikörper reagiert nicht mit der die Arg⁵⁹³→Cys-Mutation enthaltenden rekombinanten A2-Domäne. Die oben angeführten Daten zeigen, daß die Bindung von anti-Faktor VIII-Antikörpern durch selektive Modifikation der immundominanten Stellen am Faktor VIII-Protein moduliert werden kann. Ähnlich dem oben angeführten Beispiel kann die selektive Modifikation anderer immundominanter Bereiche von Faktor VIII verwendet werden, um die Bindung von anti-Faktor VIII-Antikörpern zu behindern.

### BEISPIEL 10: Formulierung der Präparation des gereinigten mutierten Proteins zu einer pharmazeutischen Präparation

Die gemäß Beispiel 2 und 9 erzeugten mutierten Proteine wurden mittels herkömmlicher Mittel, darunter chromatographische Verfahren und/oder Fällungstechniken, gereinigt. Die gereinigte Protein-Präparation wurde dann unter Verwendung von Standard-Techniken, wie Sterilfiltration und Ultra/Diafiltration, zu einer physiologisch akzeptablen Präparation formuliert. Der in der pharmazeutischen Präparation verwendete, bevorzugte Puffer ist ein physiologischer NaCl-Puffer oder ein Tris-Puffer mit einem pH-Wert im Bereich von 6-8, vorzugsweise zwischen 7 und 7,5. Die Konzentration des Proteins in der pharmazeutischen Präparation wird gemäß der zur Behandlung eines Patienten erforderlichen Dosis ausgewählt.

Die Dosis der Faktor VIII-Präparationen gemäß der Erfindung hängt von der Art, dem Ausmaß und der Dauer der blutenden Verletzung sowie von der Schwere der Hämophilie bzw. dem Inhibitor-Titer ab. Die Anfangsdosis liegt üblicherweise zwischen 1 und 500 E/kg, vorzugsweise zwischen 10 und 200 E/kg, Körpergewicht.

## Patentansprüche

1. Stabile pharmazeutische Präparation, enthaltend ein Protein mit Faktor-VIII-Prokoagulationsaktivität und vWF-Bindungsaktivität, dadurch gekennzeichnet, daß das Protein eine Aminosäuresequenz hat, die von der Aminosäuresequenz des Faktor VIII-Proteins abgeleitet ist und wobei diese abgeleitete Aminosäuresequenz mindestens eine Mutation in mindestens einer immundominanten Region von Faktor VIII aufweist.

2. Präparation nach Anspruch 1, dadurch gekennzeichnt, daß die immundominante Region die C2- und/oder die A2-Domäne ist.

3. Präparation nach Anspruch 1 oder 2, dadurch gekennzeichnet, daß das Protein eine Faktor VIII-Prokoagulations-Aktivität von mindestens 30%, vorzugsweise mindestens 50%, mehr bevorzugt mindestens 80%, insbesondere mindestens 100%, der Faktor VIII-Prokoagulations-Aktivität eines Faktor VIII-Proteins ohne diese Mutation aufweist.

4. Präparation nach einem der Ansprüche 1 bis 3, dadurch gekennzeichnet, daß das Protein eine vWF-Bindungsaktivität von mindestens 30%, vorzugsweise mindestens 50%, mehr bevorzugt mindestens 80%, insbesondere mindestens 100%, der vWF-Bindungsaktivität eines Faktor VIII-Proteins ohne diese Mutation aufweist.

5. Präparation nach einem der Ansprüche 1 bis 4, dadurch gekennzeichnet, daß das Protein die Faktor VIII-Prokoagulations-aktivität in Gegenwart eines Faktor VIII-Inhibitors aufweist.

6. Präparation nach einem der Ansprüche 1 bis 5, dadurch gekennzeichnet, daß das Protein die vWF-Bindungsaktivität in Gegenwart eines Faktor VIII-Inhibitors aufweist.

7. Präparation nach Anspruch 5 oder 6, dadurch gekennzeichnet, daß das Protein eine Aktivität in Gegenwart eines von einem Faktor VIII-Inhibitor-Patienten isolierten Faktor VIII-Inhibitors oder eines äquivalenten, durch die Hybridoma-Technologie erzeugten Antikörpers, wie CLB-CAg117, aufweist.

8. Präparation nach einem der Ansprüche 1 bis 7, dadurch gekennzeichnet, daß die immundominante Region durch einen von Faktor VIII-Inhibitor-Patienten isolierten Faktor VIII oder einem durch die Hybridoma-Technologie erzeugten äquivalenten Antikörper, wie CLB-CAg117, erkannt wird.

9. Präparation nach einem der Ansprüche 1 bis 8, dadurch gekennzeichnet, daß die Mutation ausgewählt ist aus der Gruppe umfassend die Mutationen R2307Q und R563C.

10. Präparation nach einem der Ansprüche 1 bis 9, dadurch gekennzeichnet, daß das Protein ein B-Domänen-deletiertes Faktor VIII-Protein ist, welches diese Mutation aufweist.

11. Präparation nach einem der Ansprüche 1 bis 10, dadurch gekennzeichnet, daß sie zusätzlich vWF und/oder Fragmente desselben enthält.

12. Biologisch aktiver Faktor VIII-Komplex, welcher ein Protein gemäß einem der Ansprüche 1 bis 11 und vWF oder ein Fragment desselben aufweist.

13. Verfahren zur Herstellung einer Präparation nach einem der Ansprüche 1 bis 11, dadurch gekennzeichnet, daß das Protein in Säuger-Zellen exprimiert, aus diesen Zellen gereinigt und zu einer pharmazeutischen Präparation formuliert wird.

14. Verfahren nach Anspruch 13, dadurch gekennzeichnet, daß die Expression bei Temperaturen zwischen 30 und 45°C, vorzugsweise bei Temperaturen von 25 bis 37°C, durchgeführt wird.

15. Verfahren nach Anspruch 13 oder 14, dadurch gekennzeichnet, daß das Protein mit vWF oder Fragmenten desselben co-exprimiert wird.

16. Verfahren zur Herstellung einer Präparation nach einem der Ansprüche 1 bis 11, dadurch gekennzeichnet, daß das Protein aus Plasma eines Patienten, der diese Mutation in einer immundominanten Region von Faktor VIII trägt, gereinigt und zu einer pharmazeutischen Präparation formuliert wird.

17. Verfahren nach einem der Ansprüche 13 bis 16, dadurch gekennzeichnet, daß vWF und/oder Fragmente davon während der Reinigungs- oder Formulierungsschritte zugegeben wird (werden).

18. Verwendung eines Proteins nach einem der Ansprüche 1 bis 11 zur Herstellung einer pharmazeutischen Präparation zur Behandlung von Patienten mit Faktor VIII-Inhibitoren oder Patienten mit einem Faktor-VIII-Inhibitoren-Risiko.

19. Antikörper-Präparation, dadurch gekennzeichnet, daß sie Antikörper gegen ein Protein nach einem der Ansprüche 1 bis 11 aufweist.

20. Verwendung einer Antikörper-Präparation nach Anspruch 19 zur Reinigung oder Bestimmung eines Proteins nach einem der Ansprüche 1 bis 11.
